# EUROPEAN PATENT APPLICATION

(11) **EP 4 647 084 A1**
(43) Date of publication of application: **12.11.2025**
(21) Application number: 24738561.0
(22) Date of filing: 05.01.2024
(51) Int. Cl.: A61K 39/00, A61K 9/00, A61K 38/00, A61K 39/395, A61P 11/00

(54) **METHOD FOR TREATING ASTHMA BY USING TSLP ANTIBODY**

(30) Priority: 06.01.2023 CN 202310020479; 25.09.2023 CN 202311239679
(71) Applicant: Jiangsu Hengrui Pharmaceuticals Co., Ltd., Lianyungang, Jiangsu 222047 (CN); Shanghai Shengdi Pharmaceutical Co., Ltd, Shanghai 201210 (CN)
(72) Inventor: FU, Meng, Lianyungang, Jiangsu 222047 (CN); SHEN, Kai, Lianyungang, Jiangsu 222047 (CN); LIN, Hongda, Lianyungang, Jiangsu 222047 (CN); WANG, Xiaopeng, Lianyungang, Jiangsu 222047 (CN); HAN, Huize, Lianyungang, Jiangsu 222047 (CN)
(74) Representative: Broom, Ashley John
(86) International application number: PCT/CN2024/070792
(87) International publication number: WO 2024/146630

(57) **Abstract**

The present disclosure provides a method for treating asthma using a TSLP antibody. Specifically, the present disclosure utilises an antibody against thymic stromal lymphopoietin (TSLP) or an antigen-binding fragment thereof to treat asthma and/or reduce the frequency and/or severity of asthma exacerbations, and/or reduce the annualised asthma exacerbation rate, and/or reduce the subject's ACQ-6 score.

## Description

### Technical Field

The present disclosure relates to a method for treating asthma or reducing the frequency and/or severity of asthma exacerbations, or reducing the annualised asthma exacerbation rate, or reducing the subject's ACQ-6 score using an antibody against thymic stromal lymphopoietin (TSLP) or an antigen-binding fragment thereof.

### Background Art

Asthma is a serious chronic inflammatory disease of the airway, affecting approximately 334 million people worldwide. With environmental degradation and increased air pollution, more people may suffer from this disease, posing a serious threat to human life and health.

Thymic stromal lymphopoietin (TSLP) is a cytokine produced by epithelial cells in response to pro-inflammatory stimuli, and mainly promote allergic inflammatory responses via its action on dendritic cells and mast cells. TSLP is a cytokine similar to interleukin-7 (IL-7) and was first discovered in conditioned media from murine thymic stromal cells. TSLP is predominantly expressed in the epithelial cells of the lungs, skin and intestines. TSLP consists of four alpha-helices and two loops, AB and CD, with three pairs of disulphide bonds formed by six cysteine residues, two N-glycosylation sites, and a molecular weight of approximately 15-20 kD. The TSLP receptor is a complex comprising two components: TSLPR and IL-7Rα. TSLP initially binds to TSLPR with relatively low affinity, then recruits IL-7Rα with high affinity, ultimately activating signalling pathways such as STAT5, leading to the maturation of dendritic cells (DCs) and the differentiation of T cells.

Myeloid dendritic cells (mDCs) are the primary effector cells of TSLP; TSLP acts on immature mDCs, inducing the secretion of cytokines IL-8, eotaxin-2, TARC and MDC, and upregulating OX40L expression. In the absence of IL-12, OX40L binds to naive CD4+ T cells, promoting their differentiation into Th2 cells, which subsequently secrete Th2 cytokines such as IL-5, IL-4, IL-9, IL-13 and TNF, thus inducing Th2-mediated inflammatory responses. Additionally, TSLP can induce DCs to produce IL-8, which recruits neutrophils, resulting in innate immune inflammation mediated by neutrophils. TSLP can also induce DCs to produce eotaxin-2, which recruits eosinophils; together with IL-5, this facilitates rapid eosinophilic infiltration and inflammation. TSLP also acts on mast cells and natural killer cells, mediating innate inflammation through the induction of IL-4, IL-6, IgE and other factors. In summary, TSLP can simultaneously induce both innate and Th2 inflammation, leading to increased mucus production, airway remodelling and narrowing, severe cellular fibrosis, and progressive development into asthma, atopic dermatitis, and allergic rhinitis-three major allergic diseases. Therefore, blocking TSLP is a potentially effective strategy for treating asthma, atopic dermatitis and related diseases.

Currently, only tezepelumab (TEZSPIRE), jointly developed by AstraZeneca (AZ) and Amgen, has been approved for market. In the Phase II PATHWAY study conducted in patients with uncontrolled severe asthma, the dosing protocols included a low-dose group (70 mg Q4W), a medium-dose group (210 mg Q4W), and a high-dose group (280 mg Q2W), with the final approved dosing regimen being 210 mg Q4W.

WO2020244544 discloses a novel TSLP antibody or antigen-binding fragment thereof, which is incorporated herein by reference.

### Summary of Invention

The present disclosure provides a method and pharmaceutical use for treating asthma in subjects in need, or for reducing the frequency and/or severity of asthma exacerbations, and/or reducing the annualised asthma exacerbation rate, and/or reducing the subject's ACQ-6 score; this method features a simple protocol, low dosing frequency and small dosage, effectively alleviating the treatment burden and improving patient compliance.

The present disclosure provides any one or any combination of the following methods:
1) a method for treating a subject suffering from asthma, 2) a method for reducing the frequency and/or severity of asthma exacerbations in a subject, 3) a method for reducing the annualised asthma exacerbation rate in a subject, 4) a method for reducing the ACQ-6 score of a subject. On the other hand, the present disclosure provides any one or any combination of the following uses:
1) the use of an anti-TSLP antibody or antigen-binding fragment thereof in the preparation of a medicament for treating a subject suffering from asthma;
2) the use of an anti-TSLP antibody or antigen-binding fragment thereof in the preparation of a medicament for reducing the frequency and/or severity of asthma exacerbations in a subject;
3) the use of an anti-TSLP antibody or antigen-binding fragment thereof in the preparation of a medicament for reducing the annualised asthma exacerbation rate in a subject;
4) the use of an anti-TSLP antibody or antigen-binding fragment thereof in the preparation of a medicament for reducing a subject's ACQ-6 score;
5) an anti-TSLP antibody or antigen-binding fragment thereof for treating a subject suffering from asthma;
6) an anti-TSLP antibody or antigen-binding fragment thereof for reducing the frequency and/or severity of asthma exacerbations in a subject;
7) an anti-TSLP antibody or antigen-binding fragment thereof for reducing the annualised asthma exacerbation rate in a subject;
8) an anti-TSLP antibody or antigen-binding fragment thereof for reducing a subject's ACQ-6 score.

On the other hand, the present disclosure further provides any one of the following uses of an anti-TSLP antibody or antigen-binding fragment thereof in combination with other asthma treatment drugs (e.g., background therapy for asthma):
1) use in the preparation of a medicament,
2) for use in the preparation of a medicament for treating a subject suffering from asthma, or reducing the frequency and/or severity of asthma exacerbations, or reducing the subject's ACQ-6 score.
3) for treating a subject suffering from asthma, or for reducing the frequency and/or severity of asthma exacerbations, or reducing the annualised asthma exacerbation rate, or reducing the subject's ACQ-6 score.

In some embodiments, the other asthma treatment drugs include:
a) corticosteroids, such as inhaled corticosteroids (ICS) (e.g., fluticasone propionate, budesonide, beclometasone); or oral corticosteroids (e.g., hydrocortisone, dexamethasone, prednisone);
b) bronchodilators such as long-acting β2 agonists (LABA) (e.g., vilanterol, formoterol), short-acting β2 agonists (SABA) (e.g., salbutamol, levalbuterol) and anticholinergic agents such as ipratropium bromide, tiotropium bromide, aclidinium and glycopyrronium bromide;
c) long-acting muscarinic receptor antagonists (LAMA), e.g., tiotropium, umeclidinium, glycopyrronium;
d) leukotriene receptor antagonists (LTRA), e.g., theophylline or its derivatives, montelukast, zafirlukast and pranlukast;
e) cromolyn;
f) antihistamines;
g) anti-leukotriene agents;
h PDE-4 inhibitors.

In some embodiments, the other asthma treatment drugs include: 1) inhaled corticosteroids (ICS) (e.g., fluticasone propionate, budesonide, beclometasone); and 2) at least one additional agent selected from the following group:
a) oral corticosteroids (e.g., hydrocortisone, dexamethasone, prednisone);
b) bronchodilators such as long-acting β2 agonists (LABA) (e.g., vilanterol, formoterol), short-acting β2 agonists (SABA) (e.g., salbutamol, levalbuterol) and anticholinergic agents such as ipratropium bromide, tiotropium bromide, aclidinium and glycopyrronium bromide;
c) long-acting muscarinic receptor antagonists (LAMA), e.g., tiotropium, umeclidinium, glycopyrronium;
d) leukotriene receptor antagonists (LTRA), e.g., theophylline or its derivatives, montelukast, zafirlukast and pranlukast;
e) cromolyn;
f) antihistamines;
g) anti-leukotriene agents;
h) PDE-4 inhibitors.

In some embodiments, the other asthma treatment drugs include: medium-dose ICS (e.g., fluticasone propionate, budesonide, beclometasone), such as a total daily dose >250 and ≤500 µg DPI or pMDI fluticasone propionate, or equivalent medication. In some embodiments, the other asthma treatment drugs include: high-dose ICS (e.g., fluticasone propionate, budesonide, beclometasone), such as a total daily dose >500 µg DPI or pMDI fluticasone propionate, or equivalent medication. In some embodiments, equivalent ICS dosages shall be based on the Guidelines for the Prevention and Treatment of Bronchial Asthma (2020 Edition).

In some embodiments, the use or method disclosed herein delays the time to asthma exacerbation compared with no administration of the anti-TSLP antibody or antigen-binding fragment.

In some embodiments, the use or method disclosed herein reduces the dose or frequency of administration of background therapy. In some embodiments, the background therapy includes medium- or high-dose inhaled corticosteroids (ICS) (e.g., fluticasone propionate, budesonide, beclometasone).

The term "background therapy" refers to standard or routine treatments for asthma known in the field.

In some embodiments, the method or use comprises: 1) administering at least one anti-TSLP antibody or antigen-binding fragment to a patient at dosing intervals greater than 4 weeks, wherein the dosing interval is selected from 5 weeks, 6 weeks, 7 weeks, 8 weeks, 9 weeks, 10 weeks, 12 weeks, 14 weeks, 16 weeks, 18 weeks, 20 weeks, 22 weeks, 24 weeks, 26 weeks, 28 weeks, 30 weeks, 32 weeks or more, or the dosing interval is selected from 1 month, 2 months, 3 months, 4 months, 5 months, 6 months, 7 months, 8 months, 9 months, 10 months, 11 months, 12 months or more; or 2) administering once every 10, 12, 14, 16, 18, 20, 22, 24, 26, 28 weeks, or once every 1 month, 2 months, 3 months, 4 months, 5 months, 6 months, 7 months, 8 months, 9 months, 10 months, 11 months, 12 months. In some embodiments, the method comprises: 1) a dosing interval selected from 12, 16, 20, 24 weeks or more, or a dosing interval selected from 3 months, 4 months, 5 months, 6 months or more; or 2) administration once every 12, 16, 20, 24 weeks, or once every 3 months, 4 months, 5 months, 6 months. In some embodiments, the method comprises: 1) a dosing interval selected from 12 weeks, 24 weeks or more, or a dosing interval selected from 3 months, 6 months or more; or 2) administration once every 12 weeks, 24 weeks, or once every 3 months, 6 months.

In some embodiments, the method or use comprises administering to a patient at least one anti-TSLP antibody or antigen-binding fragment at a dosage of up to 600 mg per administration, with a dosing interval of no less than 10 weeks, wherein the dosing interval is selected from 10 weeks, 12 weeks, 14 weeks, 16 weeks, 18 weeks, 20 weeks, 22 weeks, 24 weeks, 26 weeks, 28 weeks, 30 weeks, 32 weeks or more, or the dosing interval is selected from 1 month, 2 months, 3 months, 4 months, 5 months, 6 months, 7 months, 8 months, 9 months, 10 months, 11 months, 12 months or more. In some embodiments, the dosing interval is selected from 12, 16, 20, 24 weeks or more, or the dosing interval is selected from 3 months, 4 months, 5 months, 6 months or more. In some embodiments, the dosing interval is selected from 12 weeks, 24 weeks or more, or the dosing interval is selected from 3 months, 6 months or more.

In some embodiments, the dosage of the anti-TSLP antibody or antigen-binding fragment is up to about 600 mg, for example 50-600 mg, for example 50-550 mg, 50-500 mg, 50-450 mg, 50-400 mg, 100-550 mg, 100-500 mg, 100-450 mg, 100-400 mg, 150-500 mg, 150-450 mg, 150-400 mg. In some embodiments, the dosage of the anti-TSLP antibody or antigen-binding fragment is 100-500 mg or 150-450 mg. In some embodiments, the dosage of the anti-TSLP antibody or antigen-binding fragment is about 50 mg, about 100 mg, about 150 mg, about 175 mg, about 200 mg, about 225 mg, about 250 mg, about 275 mg, about 300 mg, about 325 mg, about 350 mg, about 375 mg, about 400 mg, about 450 mg, about 600 mg. In some embodiments, the dosage of the anti-TSLP antibody or antigen-binding fragment is selected from about 150 mg, about 200 mg, about 250 mg, about 300 mg, about 350 mg, about 400 mg or about 450 mg. In some embodiments, the dosage of the anti-TSLP antibody or antigen-binding fragment is about 200 mg or about 400 mg.

In some embodiments, the dosage of the anti-TSLP antibody or antigen-binding fragment is 100-500 mg; the dosing interval is selected from 12, 16, 20 or 24 weeks. In some embodiments, the dosage of the anti-TSLP antibody or antigen-binding fragment is 150-450 mg; the dosing interval is 12 weeks or 24 weeks. In some embodiments, the dosing regimen of the anti-TSLP antibody or antigen-binding fragment is selected from 200 mg Q24W, 400 mg Q24W or 400 mg Q12W.

In some embodiments, the anti-TSLP antibody or antigen-binding fragment is administered for a duration of at least 2 months, 3 months, 4 months, 5 months, 6 months, 8 months, 10 months, 12 months or longer.

In some embodiments of the present disclosure, the anti-TSLP antibody or antigen-binding fragment is administered parenterally. In some embodiments, the TSLP antibody or antigen-binding fragment is administered intravenously. In some embodiments, the TSLP antibody or antigen-binding fragment is administered subcutaneously. In some embodiments, the TSLP antibody or antigen-binding fragment is administered via subcutaneous injection. For example, an injectable form of the anti-TSLP antibody or antigen-binding fragment is an injection solution or lyophilised powder injection, comprising the anti-TSLP antibody or antigen-binding fragment, a buffer, a stabiliser and a surfactant. The buffer is a histidine-acetate system; the stabiliser may be selected from one or more of sugars, amino acids and EDTA, for example sugars such as trehalose, sucrose, sorbitol and mannitol, for example amino acids such as histidine, tryptophan and methionine, and for example EDTA. The surfactant is selected from polysorbates, such as polysorbate 80 or polysorbate 20, for example polysorbate 80. For example, the injectable form of the TSLP antibody or antigen-binding fragment comprises the anti-TSLP antibody or antigen-binding fragment, histidine-acetate buffer, sucrose and polysorbate 80. WO2022116858 relates to compositions of anti-TSLP antibodies, the full content of which is incorporated herein by reference.

In some embodiments, the subject suffers from allergic asthma or non-allergic asthma.

In some embodiments, the subject suffers from mild asthma.

In some embodiments, the subject suffers from moderate-to-severe asthma or severe asthma.

In some embodiments, the subject suffers from uncontrolled asthma. In some embodiments, the subject suffers from severe uncontrolled asthma.

In some embodiments, the subject suffers from eosinophilic asthma or non-eosinophilic asthma.

In some embodiments, the subject has a high eosinophil count at the start of treatment or at diagnosis, for example ≥250 cells/µL, for example ≥300 cells/µL.

In some embodiments, the subject has a low eosinophil count at the start of treatment or at diagnosis, for example <300 cells/µL, for example <250 cells/µL.

In some embodiments, the subject is an adult. In some embodiments, the subject is a child or adolescent.

In some embodiments, the subject exhibits low Th2 characteristics. In some embodiments, the subject exhibits Th2 characteristics of IgE ≤100 IU/mL or eosinophil count <140 cells/µL, particularly at the start of treatment or at diagnosis.

In some embodiments, the subject suffers from moderate-to-severe asthma or severe asthma with partial or uncontrolled response to background asthma therapy.

In some embodiments, the subject remains on maintenance background therapy.

In some embodiments, the background therapy includes at least one of the following:
a) corticosteroids, such as inhaled corticosteroids (ICS) (e.g., fluticasone propionate, budesonide, beclometasone); or oral corticosteroids (e.g., hydrocortisone, dexamethasone, prednisone);
b) bronchodilators such as long-acting β2 agonists (LABA) (e.g., vilanterol, formoterol), short-acting β2 agonists (SABA) (e.g., salbutamol, levalbuterol) and anticholinergic agents such as ipratropium bromide, tiotropium bromide, aclidinium and glycopyrronium bromide;
c) long-acting muscarinic receptor antagonists (LAMA), e.g., tiotropium, umeclidinium, glycopyrronium;
d) leukotriene receptor antagonists (LTRA), e.g., theophylline or its derivatives, montelukast, zafirlukast and pranlukast;
e) cromolyn;
f) antihistamines;
g) anti-leukotriene agents;
h) PDE-4 inhibitors.

In some embodiments, the background therapy includes at least one of the following:
1) inhaled corticosteroids (ICS) (e.g., fluticasone propionate, budesonide, beclometasone); and 2) at least one additional agent selected from the following group:
a) oral corticosteroids (e.g., hydrocortisone, dexamethasone, prednisone);
b) bronchodilators such as long-acting β2 agonists (LABA) (e.g., vilanterol, formoterol), short-acting β2 agonists (SABA) (e.g., salbutamol, levalbuterol) and anticholinergic agents such as ipratropium bromide, tiotropium bromide, aclidinium and glycopyrronium bromide;
c) long-acting muscarinic receptor antagonists (LAMA), e.g., tiotropium, umeclidinium, glycopyrronium;
d) leukotriene receptor antagonists (LTRA), e.g., theophylline or its derivatives, montelukast, zafirlukast and pranlukast;
e) cromolyn;
f) antihistamines;
g) anti-leukotriene agents;
h) PDE-4 inhibitors.

In some embodiments, the background therapy includes at least one of the following:
1) medium- or high-dose inhaled corticosteroids (ICS) (e.g., fluticasone propionate, budesonide, beclometasone); and 2) at least one additional agent selected from the following group:
a) oral corticosteroids (e.g., hydrocortisone, dexamethasone, prednisone);
b) bronchodilators such as long-acting β2 agonists (LABA) (e.g., vilanterol, formoterol), short-acting β2 agonists (SABA) (e.g., salbutamol, levalbuterol) and anticholinergic agents such as ipratropium bromide, tiotropium bromide, aclidinium and glycopyrronium bromide;
c) long-acting muscarinic receptor antagonists (LAMA), e.g., tiotropium, umeclidinium, glycopyrronium;
d) leukotriene receptor antagonists (LTRA), e.g., theophylline or its derivatives, montelukast, zafirlukast and pranlukast;
e) cromolyn;
f) antihistamines;
g) anti-leukotriene agents;
h) PDE-4 inhibitor;

Wherein the high-dose inhaled corticosteroid is a daily total dose of >500 µg DPI or pMDI fluticasone propionate, or an equivalent drug;

The medium-dose inhaled corticosteroid is a daily total dose of >250 and ≤500 µg DPI or pMDI fluticasone propionate, or an equivalent drug. In some embodiments, equivalent ICS dosages shall be based on the Guidelines for the Prevention and Treatment of Bronchial Asthma (2020 Edition). In some embodiments, the background therapy comprises:
a) Corticosteroids, such as medium-dose or high-dose inhaled corticosteroids (ICS);
b) In accordance with the Guidelines for the Prevention and Treatment of Bronchial Asthma (2020 edition), combined with at least one other asthma control medication, selected from long-acting β2 receptor agonists (LABA), long-acting cholinergic receptor antagonists (LAMA), leukotriene receptor antagonists (LTRA), or oral corticosteroids (OCS).

In some embodiments, the background therapy comprises: medium-dose ICS (e.g., fluticasone propionate, budesonide, beclometasone), such as a total daily dose >250 and ≤500 µg DPI or pMDI fluticasone propionate, or equivalent medication.

In some embodiments, the background therapy comprises: high-dose ICS (e.g., fluticasone propionate, budesonide, beclometasone), such as a total daily dose >500 µg DPI or pMDI fluticasone propionate, or equivalent medication.

In some embodiments, the present method delays the time to asthma exacerbation compared with no administration of the anti-TSLP antibody or its antigen-binding fragment.

In some embodiments, the present method reduces the dosage or frequency of administration of background therapy. In some embodiments, the background therapy includes medium- or high-dose inhaled corticosteroids (ICS) (e.g., fluticasone propionate, budesonide, beclometasone).

In some embodiments, the subject is selected according to at least one of the following criteria:
a) Prior to administration of the antibody or antibody-binding fragment, the subject has a pre-bronchodilator FEV₁ of ≥40% and <80% of the predicted value;
b) Prior to administration of the antibody or antibody-binding fragment, the subject has an ACQ-6 score ≥1.5.

In some embodiments, at Week 24 or Week 48 of treatment, the subject achieves at least one of the following:
a) Improved annualised asthma exacerbation rate (AAER);
b) Improved forced vital capacity (FVC);
c) Improved forced expiratory volume in one second (FEV1);
d) Improved peak expiratory flow (PEF);
e) Improved fractional exhaled nitric oxide (FeNO), blood eosinophil count (EOS), and/or serum immunoglobulin (IgE);
f) Decreased ACQ-6 score;
g) Improved Asthma Quality of Life Questionnaire (AQLQ) score;
h) Improved asthma symptom diary score;
i) Reduced use of rescue medication;
j) Reduced mean number of nocturnal awakenings;
k) Reduced AAER resulting in hospitalisation at Week 48;
l) Improved time to first acute asthma attack;
m) Improvement in SNOT-22;
n) Improvement in incidence of CompEx (composite exacerbation endpoint).

In some embodiments, the method reduces eosinophils in the subject's blood, sputum, bronchoalveolar lavage fluid or lungs.

In some embodiments, the method reduces Th2 cell count, e.g., converting the subject's cell count from Th2-high to Th2-low population.

In another aspect, the present disclosure provides a method for treating asthma in a subject or reducing the frequency and/or severity of asthma exacerbation, and/or reducing annualised asthma exacerbation rate, and/or reducing the subject's ACQ-6 score, comprising administering a therapeutically effective amount of an anti-TSLP antibody or antigen-binding fragment thereof, wherein the subject has non-eosinophilic or low-eosinophilic features.

In another aspect, the present disclosure provides a method for treating asthma in a subject or reducing the frequency and/or severity of asthma exacerbation, or reducing annualised asthma exacerbation rate, or reducing the subject's ACQ-6 score, comprising administering a therapeutically effective amount of an anti-TSLP antibody or antigen-binding fragment thereof, wherein the subject has low Th2 features.

In another aspect, the present disclosure provides an anti-TSLP antibody or antigen-binding fragment thereof for use in treating asthma in a subject, and/or reducing the frequency and/or severity of asthma exacerbation, and/or reducing annualised asthma exacerbation rate, and/or reducing the subject's ACQ-6 score; wherein the subject has low Th2 features.

In some embodiments, the anti-TSLP antibody or antigen-binding fragment is any of the anti-TSLP antibodies or antigen-binding fragments disclosed in WO2020244544A1 or WO2022116858A1, which are hereby incorporated by reference in their entirety.

In some embodiments, the anti-TSLP antibody or antigen-binding fragment comprises a heavy chain variable region and a light chain variable region, wherein:
the heavy chain variable region comprises HCDR1, HCDR2 and HCDR3 as shown in SEQ ID NO: 1, SEQ ID NO: 2 and SEQ ID NO: 3, respectively; and
the light chain variable region comprises LCDR1, LCDR2 and LCDR3 as shown in SEQ ID NO: 4, SEQ ID NO: 5 and SEQ ID NO: 6, respectively.

Wherein the aforementioned CDR sequences are listed in the table below:

| | | | |
|---|---|---|---|
| HCDR1 | NYLIE SEQ ID NO: 1 | LCDR1 | KASQSVSSDVT SEQ ID NO: 4 |
| HCDR2-N55V | VIDPGVGDTNYNE NFKG SEQ ID NO: 2 | LCDR2-N53E | YVSEHYT SEQ ID NO: 5 |
| HCDR3 | EDNTGTAFDY SEQ ID NO: 3 | LCDR3 | QQHHRFPLT SEQ ID NO: 6 |

In some embodiments, the anti-TSLP antibody is a murine antibody, a chimeric antibody, or a humanised antibody.

In optional embodiments, the light and heavy chain framework region sequences on the variable regions of the humanised antibody light and heavy chains are human germline light and heavy chain framework region sequences or mutant sequences thereof.

In some embodiments, the anti-TSLP antibody comprises a heavy chain variable region and a light chain variable region, wherein the heavy chain variable region comprises an amino acid sequence as shown in SEQ ID NO: 7 or an amino acid sequence having at least 90% identity thereto, and the light chain variable region comprises an amino acid sequence as shown in SEQ ID NO: 8 or an amino acid sequence having at least 90% identity thereto.

Heavy chain variable region

EVQLVQSGAEVKKPGSSVKVSCKASGYTFSNYLIEWVRQAPGQGLEWIGV
IDPGVGDTNYNENFKGRATLTADKSTSTAYIELSSLRSEDTAVYYCAREDNTGTA
FDYWGQGTTVTVSS

Note: Single underlined sections indicate CDRs and double underlined sections indicate back mutation sites.
SEQ ID NO: 7

In some embodiments, the anti-TSLP antibody or its antigen-binding fragment further comprises an antibody constant region; for example, the heavy chain constant region of the antibody constant region is selected from human IgG1, IgG2, IgG3 and IgG4 constant regions and their conventional variants; the light chain constant region of the antibody constant region is selected from human antibody κ and λ chain constant regions and their conventional variants.

Light chain variable region

SIVMTQTPLSLSVTPGQPASISCKASQSVSSDVTWYLQKPGQSPQLLIYYVSE
HYTGVPDRFSGSGYGTDFTLKISRVEAEDVGVYYCQQHHRFPLTFGQGTKLEIK
SEQ ID NO: 8

In some embodiments, the anti-TSLP antibody comprises a heavy chain and a light chain, wherein the heavy chain comprises an amino acid sequence as shown in SEQ ID NO: 9 or an amino acid sequence having at least 90% identity thereto, and the light chain comprises an amino acid sequence as shown in SEQ ID NO: 10 or an amino acid sequence having at least 90% identity thereto.

Heavy chain

EVQLVQSGAEVKKPGSSVKVSCKASGYTFSNYLIEWVRQAPGQGLEWIGVI
DPGVGDTNYNENFKGRATLTADKSTSTAYIELSSLRSEDTAVYYCAREDNTGTAF
DYWGQGTTVTVSSASTKGPSVFPLAPSSKSTSGGTAALGCLVKDYFPEPVTVSWNSG

ALTSGVHTFPAVLQSSGLYSLSSVVTVPSSSLGTQTYICNVNHKPSNTKVDKKVEPKSCD
KTHTCPPCPAPELLGGPSVFLFPPKPKDTLYITREPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTP
PVLDSDGSFFLYSKLTVDKSRWQQGNVFSCSVMHEALHNHYTQKSLSLSPGK
SEQ ID NO: 9

Light chain

SIVMTQTPLSLSVTPGQPASISCKASQSVSSDVTWYLQKPGQSPQLLIYYVS
EHYTGVPDRFSGSGYGTDFTLKISRVEAEDVGVYYCQQHHRFPLTFGQGTKLEI
KRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 10

In certain embodiments, the anti-TSLP antibody or antigen-binding fragment thereof comprises a heavy chain sequence having at least 85%, 86%, 87%, 88%, 89%, 90%, 91%, 92%, 93%, 94%, 95%, 96%, 97%, 98%, or 99% sequence identity to the amino acid sequence of SEQ ID NO: 9, and a light chain sequence having at least 85%, 86%, 87%, 88%, 89%, 90%, 91%, 92%, 93%, 94%, 95%, 96%, 97%, 98%, or 99% sequence identity to the amino acid sequence of SEQ ID NO: 10.

Unless otherwise defined, the terms used in this disclosure have the following meanings:
Unless the context clearly requires otherwise, throughout the specification and claims, the terms "comprising," "having," "including," and the like shall be understood to have an inclusive rather than exclusive or exhaustive meaning; that is, they shall be interpreted as meaning "including but not limited to."

The term "and/or", for example "X and/or Y", shall be understood to mean "X and Y" or "X or Y" and is intended to explicitly support either or both interpretations.

The term "thymic stromal lymphopoietin (TSLP)" refers to a type I four-α-helix bundle cytokine, which is an epithelial-cell-derived cytokine produced in response to pro-inflammatory stimuli, closely related to interleukin-7 (IL-7), and initiates allergic reactions by stimulating dendritic cells (DCs). It is an important factor in regulating human immune responses. The term "TSLP" includes variants, isoforms, homologues, orthologues and paralogues of TSLP.

"Buffer" refers to a buffer that can withstand pH changes due to the effect of acid-base conjugate components. The examples of buffers that control pH within an appropriate range include acetate, succinate, gluconate, histidine, oxalate, lactate, phosphate, citrate, tartrate, fumarate, glycylglycine and other organic acid buffers.

"Histidine buffer" is a buffer that contains histidine ions. Examples of histidinate buffers include histidine-hydrochloride, histidine-acetate, histidine-phosphate and histidine-sulphate buffers, preferably histidine-acetate buffer or histidine-hydrochloride buffer; histidine-acetate buffer is prepared from histidine and acetic acid; histidine-hydrochloride buffer is prepared from histidine and hydrochloric acid.

"Stabiliser" refers to a component that helps to maintain the structural integrity of a biopharmaceutical product, particularly during freezing and/or lyophilisation and/or storage (especially when exposed to stress). This stabilising effect can arise for various reasons and typically such stabilisers act as osmolytes that mitigate protein denaturation. As used herein, stabilisers include sugars, amino acids and EDTA; the amino acids included as stabilisers refer to those added separately, in addition to any amino acids in the buffer.

"Surfactant" refers to a surface-active agent, preferably a non-ionic surfactant. The use of surfactants can reduce protein aggregation and/or the formation of particulates in the formulation. The amount of surfactant added is such that it can reduce protein aggregation in the formulation and minimise particulate formation. The surfactants disclosed herein may be selected from polysorbates (including but not limited to polysorbate 20 and polysorbate 80), polyhydroxyalkanes, Triton, sodium dodecyl sulfate, sodium lauryl sulfate, sodium octyl glucoside, lauryl sulfobetaine, myristyl sulfobetaine, linoleyl sulfobetaine, stearyl sulfobetaine, lauroyl sarcosinate, myristoyl sarcosinate, linoleoyl sarcosinate, stearoyl sarcosinate, linoleyl betaine, myristyl betaine, cetyl betaine, lauramidopropyl betaine, cocamidopropyl betaine, linoleamidopropyl betaine, myristamidopropyl betaine, palmitamidopropyl betaine, isostearamidopropyl betaine, myristamidopropyl dimethylamine, palmitamidopropyl dimethylamine, isostearamidopropyl dimethylamine, sodium methyl cocoyl taurate, sodium methyl oleoyl taurate, polyethylene glycol, polypropylene glycol and copolymers of ethylene glycol and propylene glycol, among others. The preferred surfactant is polysorbate 80 or polysorbate 20 and more preferably polysorbate 80.

The terms "about" and "approximately" as used herein refer to values within an acceptable error range as determined by one of ordinary skill in the art, which may depend in part on how the value is measured or determined (i.e., the limitations of the measurement system). For instance, "about" in each embodiment in this field may mean that the standard deviation is within 1 or more than 1. Alternatively, "about" or "substantially comprises" may mean within ±10% of the stated value. Moreover, particularly for biological systems or processes, this term can mean up to an order of magnitude or up to five times a value. Unless otherwise specified, when a specific value appears in this application and claims, "about" or "substantially contain" should mean the specific value is assumed to be within the acceptable margin of error.

Although the disclosure provides content ranges or content values, those skilled in the art will understand that such content ranges or values encompass acceptable error margins of the measured values.

"Pharmaceutical composition" refers to a mixture containing one or more antibody-drug conjugate(s) described herein or their physiologically/pharmaceutically acceptable salts or prodrugs along with other chemical components, such as physiologically/pharmaceutically acceptable carriers and excipients. The purpose of pharmaceutical composition is to maintain the stability of active antibody component, facilitate the administration to biological organisms, and promote the absorption of active component to exert their biological activity.

The term "combination" as used in this disclosure refers to a method of administration, which means administering at least one dose of an anti-TSLP antibody or its antigen-binding fragment and another asthma therapeutic within a specified period, wherein both drugs exhibit pharmacological activity. The specified period may be within one administration cycle, for example within 12 weeks, 16 weeks, 20 weeks, or 24 weeks. The anti-TSLP antibody or its antigen-binding fragment and the other asthma therapeutic may be administered simultaneously or in no particular order. The period includes such treatment where the anti-TSLP antibody or its antigen-binding fragment and the other asthma therapeutic are administered via the same or different administration routes.

In the present disclosure, "pharmaceutical composition" and "formulation" are not mutually exclusive.

The solution form of the pharmaceutical composition described in the present disclosure contains water as solvent unless otherwise specified.

"Lyophilised formulation" refers to the formulation or pharmaceutical composition obtained after vacuum lyophilisation steps from a liquid or solution form of the pharmaceutical composition or liquid or solution formulation.

The three-letter and one-letter codes for amino acids used in this disclosure are as defined in J. Biol. Chem, 243, p3558 (1968).

The term "antibody" as used in this disclosure is used in its broadest sense, encompassing various antibody structures, including but not limited to monoclonal antibodies, polyclonal antibodies, multispecific antibodies (e.g., bispecific antibodies), full-length antibodies, or their antigen-binding fragments (also referred to as "antigen-binding portions"), provided they exhibit the desired antigen-binding activity. A full-length antibody is an immunoglobulin (Ig) comprising at least two heavy chains and two light chains joined by disulphide bonds. According to the amino acid composition and arrangement of the immunoglobulin heavy chain constant region, immunoglobulins can be classified into five types, or isotypes, namely IgM, IgD, IgG, IgA and IgE, with corresponding heavy chains of µ, δ, γ, α and ε. Each class of Ig can be further subdivided into subclasses based on differences in hinge region amino acid composition and the number and position of disulphide bonds in the heavy chain, for example, IgG can be divided into IgG1, IgG2, IgG3 and IgG4. Light chains are classified as κ or λ based on their constant regions. Each of the five classes of Ig can contain either a κ or λ light chain.

The term "variable region" or "variable domain" refers to the domain in the antibody heavy or light chain involved in antigen binding. VH and VL each contain four conserved framework regions (FRs) and three complementarity-determining regions (CDRs). The term "complementarity-determining region" or "CDR" refers to the regions within the variable domain primarily responsible for antigen binding; "framework" or "FR" refers to the variable domain residues outside of the CDRs. VH contains three CDRs: HCDR1, HCDR2 and HCDR3; VL contains three CDRs: LCDR1, LCDR2 and LCDR3. Each VH and VL consists of three CDRs and four FRs arranged sequentially from the amino terminus to the carboxy terminus in the following order: FR1, CDR1, FR2, CDR2, FR3, CDR3, FR4. A single VH or VL may be sufficient to confer antigen-binding specificity.

Various well-known methods can be used to determine the amino acid sequence boundaries of the CDRs, for example: The "Kabat" numbering scheme (see Kabat et al. (1991), "Sequences of Proteins of Immunological Interest", 5th Ed., Public Health Service, National Institutes of Health, Bethesda, MD), the "Chothia" numbering scheme, the "ABM" numbering scheme, the "contact" numbering scheme (see Martin, A.C.R. Protein Sequence and Structure Analysis of Antibody Variable Domains[J]. 2001) and the ImMunoGenTics (IMGT) numbering scheme (Lefranc, M.P. et al., Dev Comp. Immunol., 27, 55-77 (2003); Front Immunol. 2018 Oct 16;9:2278). The relationships among the different numbering schemes are well known to those skilled in the art and are shown in Table 1 below.

**Table 1 Correspondence between CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise specified, the variable region and CDR sequences in the embodiments of this disclosure follow the "Kabat" numbering scheme.

The terms "antigen-binding fragment", "functional fragment", or "antigen-binding portion" refer to one or more fragments of a full antibody that retain the ability to specifically bind to an antigen. It has been shown that fragments of a full-length antibody can be utilised to perform antigen-binding functions. Exemplary binding fragments encompassed by the term "antigen-binding fragment" include: (i) Fab fragment, a monovalent fragment comprising the VL, VH, CL and CH1 domains; (ii) F(ab')₂ fragment, a bivalent fragment comprising two Fab fragments linked via disulphide bridges in the hinge region; (iii) Fd fragment, comprising the VH and CH1 domains; (iv) Fv fragment, comprising the VH and VL domains of a single antibody arm; (v) dsFv, a stabilised antigen-binding fragment in which VH and VL are linked by an inter-chain disulphide bond; (vi) scFv; (vii) diabodies, bispecific and multispecific antibodies comprising scFv, dsFv, Fab and other fragments.

The terms "single-chain antibody", "single-chain Fv", or "scFv" refer to molecules comprising an antibody heavy chain variable domain (or region, VH) and an antibody light chain variable domain (or region, VL) linked by a linker. Such scFv molecules may generally have the structure: NH₂-VL-linker-VH-COOH or NH₂-VH-linker-VL-COOH. Numerous linkers suitable for connecting antibodies VH and VL are known in the art, such as those composed of repeated GGGGS amino acid sequences or variants thereof, for example using 1-4 repeats of such variants (Holliger et al. (1993), Proc. Natl. Acad. Sci. USA90: 6444-6448). Other linkers useful in this disclosure are described by Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56 and Roovers et al. (2001), Cancer Immunol.

A diabody is an antibody fragment in which scFv or Fab is dimerised, providing bivalent antigen-binding activity. In bivalent antigen-binding activity, the two antigens may be the same or different.

Bispecific and multispecific antibodies refer to antibodies capable of binding simultaneously to two or more antigens or epitopes, comprising an scFv or Fab fragment capable of binding TSLP.

The "conventional variants" of the human antibody heavy chain constant region and human antibody light chain constant region as described in this disclosure refer to variants of human origin disclosed in the prior art that do not alter the structure and function of the antibody variable regions, with exemplary variants including IgG1, IgG2, IgG3 or IgG4 heavy chain constant region variants bearing point mutations and amino acid substitutions, such as the known YTE mutation, L234A and/or L235A mutations, S228P mutation, and/or mutations yielding a knob-into-hole structure (so that the antibody heavy chain comprises a knob-Fc and hole-Fc combination), all of which have been shown to confer novel properties on the antibody without altering variable region function.

The terms "full-length antibody", "intact antibody", "complete antibody", and "whole antibody" are used interchangeably herein to refer to antibodies in substantially complete form, distinguishable from the antigen-binding fragments defined below. This term specifically refers to antibodies comprising constant regions on both the heavy and light chains.

Methods for producing and purifying antibodies and antigen-binding fragments are well known in the art, such as those described in Chapters 5-8 and 15 of the Cold Spring Harbor Antibody Laboratory Manual. For example, mice may be immunised with human TSLP or a fragment thereof, and the resulting antibodies can be refolded, purified, and sequenced using standard methods. Antigen-binding fragments can similarly be prepared by conventional methods. The antibodies or antigen-binding fragments described in this disclosure are engineered by adding one or more human framework (FR) regions to non-human CDR regions. Human germline FR sequences may be selected by aligning sequences in the IMGT human antibody variable region germline gene database using MOE software from the ImMunoGeneTics (IMGT) website (http://imgt.cines.fr) or obtained from the Immunoglobulin journal (2001ISBN012441351).

The engineered antibodies or antigen-binding fragments of this disclosure may be produced and purified by standard methods. For example, cDNA sequences encoding the heavy and light chains can be cloned and recombined into GS expression vectors. The recombinant immunoglobulin expression vectors can be stably transfected into CHO cells. As a more preferred prior art, mammalian expression systems result in glycosylation of antibodies, particularly at the highly conserved N-terminal sites in the Fc region. Stable clones are obtained by expressing antibodies that specifically bind to human TSLP. Positive clones are expanded in serum-free media in bioreactors to produce antibodies. The culture medium containing the secreted antibodies can be purified using conventional techniques. For example, purification can be performed using A or G Sepharose FF columns containing adjusted buffers. Non-specifically bound components are washed away. The bound antibodies can then be eluted using the pH gradient method, with antibody fragments detected by sodium dodecyl sulfate polyacrylamide gel electrophoresis (SDS-PAGE) and collected. Antibodies can be filtered and concentrated using conventional methods. Soluble mixtures and polymers can also be removed using conventional methods such as molecular sieve and ion exchange. The resulting product should be immediately frozen, for example, at -70°C, or lyophilised.

The terms "administering", "delivery" and "treatment", when applied to an animal, human, study subject, cell, tissue, organ, or biological fluid, refer to the contact of an exogenous drug, therapeutic agent, diagnostic agent, or composition with the animal, human, subject, cell, tissue, organ, or biological fluid. The terms "administering", "delivery" and "treatment" may refer to, for example, therapeutic, pharmacokinetic, diagnostic, research and experimental methods. The treatment of cells includes the contact between reagents and cells, and between reagents and fluids, where the fluids are in contact with cells. The terms "administering", "delivery" and "treatment" also refer to the application of an agent, diagnostic, binding composition, or another type of ex vivo or in vitro manipulation, for example, of cells. "Treatment", when applied to humans, veterinary or research subjects, refers to therapeutic treatments, preventive or prophylactic measures, research and diagnostic applications.

"Treatment" means administration of therapeutic agents for internal or topical use to a patient, such as a composition containing any of the conjugated compounds in the present disclosure, where the patient has one or more disease symptoms and the therapeutic agents are known to have a therapeutic effect on those symptoms. Typically, a therapeutic agent is administered in an amount effective to alleviate one or more disease symptoms in a patient or population under treatment, in order to induce regression of such symptoms or inhibit their progression to any clinically measurable extent. The amount of the therapeutic agent that effectively alleviates any specific disease symptom (also referred to as "therapeutically effective amount") may vary depending on several factors, such as the patient's disease state, age and body weight, as well as the drug's ability to produce the desired effect to the patient. Whether the disease symptom has alleviated can be evaluated by any clinical testing method commonly used by physicians or other healthcare professionals to evaluate the severity or progression of the symptom. Although the embodiments (such as therapeutic methods or products) in the present disclosure may be ineffective in alleviating each targeted disease symptom, yet any statistical testing method known in the field, such as Student's t-test, Chi-square test, Mann-Whitney U test, Kruskal- Wallis test (H-test), Jonckheere-Terpstra test and Wilcoxon test, can determine that they should alleviate the targeted disease symptoms in a statistically significant number of patients.

The term "effective amount" or "effective dose" refers to the amount of a drug, compound, or pharmaceutical composition necessary to achieve any one or more beneficial or desired therapeutic outcomes. For prophylactic purposes, beneficial or desired outcomes include eliminating or reducing risk, mitigating severity, or delaying the onset of conditions, including symptoms of the condition, its complications, and biochemical, histological, and/or behavioural manifestations of intermediate pathological phenotypes arising during disease progression. For therapeutic applications, beneficial or desired outcomes include clinical outcomes such as reducing the incidence of a condition associated with the target antigen of the disclosure or improving one or more symptoms of the condition, reducing the dosage of other agents required to treat the condition, enhancing the efficacy of another agent, and/or delaying progression of the condition associated with the target antigen of the disclosure in a patient.

"Homology" refers to sequence similarity between two polynucleotide sequences or between two polypeptides. When positions in the two sequences under comparison are occupied by identical bases or amino acid subunit monomers, for example, where both DNA molecules have an adenine at the same position, the molecules are homologous at that position. The percentage of homology between two sequences is the function of the number of matching or homologous positions shared by the two sequences divided by the number of positions compared, multiplied by 100. For example, if 6 out of 10 positions in the best alignment of two sequences are matching or homologous, the sequences are 60% homologous; if 95 out of 100 positions match, they are 95% homologous. Typically, when comparing two sequences, the alignment yielding the highest percentage of homology is used. For example, comparisons may be performed using the BLAST algorithm, where the algorithm parameters are chosen to provide the highest alignment across the entire length of each reference sequence. The following references relate to BLAST algorithms frequently used for sequence analysis: BLAST ALGORITHMS: Altschul, S.F. et al., (1990) J. Mol. Biol. 215:403-410; Gish, W. et al., (1993) Nature Genet. 3:266-272; Madden, T.L. et al., (1996) Meth. Enzymol. 266:131-141; Altschul, S.F. et al., (1997) Nucleic Acids Res. 25:3389-3402; Zhang, J. et al., (1997) Genome Res. 7:649-656. Other standard BLAST algorithms such as those provided by NCBI BLAST are also well known to those skilled in the art.

"Optional" or "optionally" means that the event or circumstance described subsequently may or may not occur, and the description includes situations where the event or circumstance occurs or does not occur.

"Asthma" refers to a common and potentially serious chronic disorder of the airways characterised by airway inflammation and constriction leading to symptoms such as wheezing, shortness of breath, chest tightness, and coughing, which vary over time in occurrence, frequency, intensity and response to therapy. As used herein, the term "asthma' refers to all phenotypes and endotypes of asthma across all severity levels and irrespective of disease control status, including non-allergic asthma, allergic asthma, mild asthma, moderate asthma, severe asthma, moderate-to-severe asthma, uncontrolled asthma, non-eosinophilic asthma, low-eosinophilic asthma, high-eosinophilic (eosinophilic) asthma, bronchial asthma, exercise-induced asthma, occupational asthma and asthma triggered by bacterial or viral infections.

"Acute asthma exacerbation" refers to the progressive worsening of asthma symptoms such as shortness of breath, coughing, wheezing and chest tightness, accompanied by a progressive decline in lung function that necessitates a change in treatment. Subjects who die due to worsening asthma symptoms are also included under acute asthma exacerbation. Such treatment changes may include any of the following:1) Treatment with systemic corticosteroids or an increase in dose for subjects already on systemic corticosteroid maintenance therapy for ≥3 days; 2) Treatment with systemic corticosteroids and an asthma event requiring emergency/urgent care or hospitalisation. Other measures associated with acute asthma attack are also being investigated to determine impact, including but not limited to worsening asthma symptoms, decline in lung function from baseline, or increased use of rescue medication. annualised asthma exacerbation rate (AAER) = Number of acute asthma attacks * 365.25 / duration of follow-up (days).

"Asthma exacerbation" refers to an acute or subacute deterioration in asthma symptoms and lung function from the patient's usual state, which may result in any of the following: Use of systemic corticosteroids for at least 3 consecutive days; a single long-acting injectable dose of corticosteroid considered equivalent to a 3-day course of systemic corticosteroids; for subjects receiving maintenance OCS, a temporary doubling of the maintenance dose for at least 3 days; an ED visit due to asthma requiring systemic corticosteroids (as described above); hospitalisation due to asthma. Other measures related to asthma exacerbation are also being investigated to determine impact. These measurements include hospitalisation related to asthma exacerbation (i.e. severe asthma exacerbation), time to first asthma exacerbation, and the proportion of subjects experiencing one or more asthma exacerbation/severe asthma exacerbation events. Severe asthma exacerbation is typically defined as requiring systemic corticosteroids or increasing the maintenance dose of oral corticosteroids for at least three days and/or emergency visits, hospitalisation, or death due to asthma. Moderate exacerbation is typically defined as events requiring treatment changes to prevent progression to severe exacerbation and the presence of one or more of the following: increased asthma symptoms, increased use of rescue medication, or decline in lung function, lasting for two or more days but not severe enough to warrant systemic corticosteroids or hospitalisation.

"Allergic asthma" refers to asthma triggered by one or more inhaled allergens. Such patients have positive IgE fluorescent enzyme immunoassay (FEIA) levels for one or more allergens that induce asthma responses. Typically, most allergic asthma is associated with Th2-type inflammation.

The term "Th2-type inflammation" refers to subjects with screening blood eosinophil counts ≥140 cells/µL and screening total serum IgE levels >100 IU/mL (Corren et al., N Engl J Med. 22;365 (f 2):1088-98, 2011). The "high Th2" asthma population or characteristic refers to subjects with IgE >100 IU/mL and blood eosinophil count ≥140 cells/µL. The "low Th2" asthma population refers to subjects with IgE <100 IU/mL and blood eosinophil count <140 cells/µL.

"IgE" refers to immunoglobulin E, which is well known to those skilled in the art. IgE is measured in international units per millilitre (IU/mL), as disclosed for example in Seagroatt and Anderson (1981).

"Non-allergic asthma" refers to patients with low eosinophil counts, low Th2, or low IgE at diagnosis. Typically, patients with "non-allergic asthma" have negative responses in IgE fluorescent enzyme immunoassays (FEIA) for a panel of allergens, including region-specific allergens. In addition to low IgE, these patients generally have low or absent eosinophil counts and low Th2 counts at diagnosis.

Eosinophil count is a blood test that measures the number of white blood cells called eosinophils. Typically, blood is drawn from a vein in the inner elbow or the back of the hand, or by pricking the skin using a lancet-type instrument. The blood is placed in a small glass tube, or onto a slide or test strip. In the laboratory, the blood is placed on a microscope slide and dye is added to the sample. This causes eosinophils to appear as orange-red granules, enabling the enumeration of eosinophils present in a specific volume of blood (e.g., one microlitre (µL)).

"Non-eosinophilic asthma" refers to asthma in which the patient does not have an eosinophil count. "Low-eosinophilic asthma" refers to asthma in which the patient's screening blood eosinophil count is <300 cells/µL. "High-eosinophilic asthma" or 'eosinophilic asthma' refers to asthma in which the patient's screening blood eosinophil count is ≥300 cells/µL.

"Mild asthma" refers to asthma that can be well controlled with GINA step 1 or step 2 treatment, i.e., only with as-needed controller medication, or with low-intensity maintenance controller therapy (e.g., low-dose ICS, leukotriene receptor antagonists, or cromones) (Global Strategy for Asthma Management and Prevention, GINA Report 2019, p.35). "Moderate asthma" refers to asthma that can be well controlled with GINA step 3 treatment, e.g., low-dose ICS-LABA (Global Strategy for Asthma Management and Prevention, GINA Report 2019, p.35). "Severe asthma" refers to asthma that requires GINA step 4 or 5 treatment, such as high-dose ICS-LABA, to prevent it from becoming "uncontrolled", or which remains "uncontrolled" despite such treatment (Global Strategy for Asthma Management and Prevention, GINA Report 2019, p.35). "Moderate-to-severe asthma" refers to both moderate and severe asthma.

"Uncontrolled asthma" refers to asthma that remains uncontrolled on GINA step 4 or 5 treatment, e.g., asthma with poor symptom control.

Asthma severity can be retrospectively assessed based on the level of treatment required to control symptoms and prevent exacerbations. After several months of controller treatment, assessment may be made and, where appropriate, step-down therapy may be attempted to determine the minimum effective level of treatment for the patient. Asthma severity is not a static characteristic and may change over months or years.

"Adult" refers to subjects aged ≥18 years. As used herein, the term "adolescent" refers to subjects aged ≥12 and <18 years. In some embodiments of the present invention, the subject is a child. As used herein, the term "child" refers to subjects aged <12 years.

Details of one or more embodiments of the present disclosure have been presented in the foregoing description. Although any methods and materials similar or equivalent to those described herein can be used in the practice or testing of the present disclosure, the preferred methods and materials are described below. Other features, objectives and advantages of the disclosure will be apparent from the specification and claims. In the specification and claims, unless the context clearly dictates otherwise, singular forms include the plural referents. Unless otherwise defined, all technical and scientific terms used herein have the same meaning as commonly understood by one of ordinary skill in the art to which this disclosure belongs. All patents and publications cited in this specification are incorporated herein by reference. The following examples are provided to more fully illustrate the preferred embodiments of the present disclosure. These examples are not to be construed as limiting the scope of the disclosure in any way, which is defined by the claims.

Abbreviations:
FEV1: Forced Expiratory Volume in 1 Second
ePRO: electronic Patient-Reported Outcome
Pre/Post-BD: Before/After use of Bronchodilator
eGFR: estimated Glomerular Filtration Rate

### Specific Embodiments

The following specific embodiments or experimental examples are provided to explain this disclosure in further detail, and are intended only for illustrative purposes and not to limit the scope of this disclosure.

Unless otherwise specified, the experimental methods in the embodiments are carried out under conventional conditions, or under the conditions recommended by the manufacturers of materials or reagents. The reagents of which the specific source is not indicated are commercially available conventional reagents.

### Example 1: Pharmacokinetic study of anti-TSLP antibody in cynomolgus monkeys

1) Test article: Anti-TSLP antibody, prepared according to the method described in WO2022116858, referred to as the hu179-33 antibody, with heavy and light chain sequences as shown in SEQ ID NO:9 and 10 in this disclosure.
2) Test method: Six cynomolgus monkeys (three males and three females) were selected and administered a single subcutaneous injection of 3 mg/kg of anti-TSLP antibody. Whole blood samples were collected and serum separated at pre-dose, and at 2 h, 8 h, 24 h, 48 h, 72 h, 96 h, 120 h (D6), 168 h (D8), 336 h (D15), 504 h (D22), 672 h (D29), 840 h (D36), 1008 h (D43), 1176 h (D50), 1344 h (D57), 1512 h (D64), 1680 h (D71), 1848 h (D78) and 2016 h (D85) post-dose. Serum drug concentrations were determined using ELISA. Pharmacokinetic parameters including t1/2 (half-life), Tₘₐₓ (time to maximum concentration), Cₘₐₓ (maximum concentration) and AUC (area under the concentration-time curve) were calculated using a WinNonLin non-compartmental analysis (NCA) model. Means and standard deviations were calculated using Waston. The experimental results are shown in Table 1, with data presented as mean ± standard deviation.

**Table 1: Pharmacokinetic parameter statistics of anti-TSLP antibody**

| Group/Dose (mg/kg) | t_{1/2} (h) | Tₘₐₓ (h) | Cₘₐₓ (µg/mL) | AUCₗₐₛₜ (h·mg/mL) | AUC_{inf} (h·mg/mL) |
|---|---|---|---|---|---|
| Anti-TSLP antibody (3 mg/kg) | 566±163 | 60.0±20.1 | 45.4±3.62 | 24.3±6.39 | 25.9±7.29 |

### Example 2: Pharmacokinetic study of anti-TSLP antibody (reference antibody) in cynomolgus monkeys

1) Test article: Tezepelumab was used as the reference antibody, prepared in-house.
2) Test method: Three male cynomolgus monkeys were selected, receiving a single subcutaneous injection of 3 mg/kg of the reference antibody tezepelumab. Whole blood was collected and serum isolated at pre-dose and at 2 h, 8 h, 24 h (D2), 48 h (D3), 72 h (D4), 96 h (D5), 120 h (D6), 168 h (D8), 336 h (D15), 504 h (D22) and 672 h (D29) post-dosing. The concentration of the reference antibody in serum samples was determined by ELISA. Pharmacokinetic parameters t_{1/2}, Tₘₐₓ, Cₘₐₓ and AUC were calculated for each animal using the WinNonlin non-compartmental analysis (NCA) model. The experimental results are shown in Table 2, with data presented as mean ± standard deviation.

**Table 2: Pharmacokinetic parameters of reference anti-TSLP antibody**

| Group/Dose (mg/kg) | t_{1/2} (h) | Tₘₐₓ (h) | Cₘₐₓ (µg/mL) | AUCₗₐₛₜ (h·mg/mL) |
|---|---|---|---|---|
| Reference antibody (3 mg/kg) | 172±77.6 | 64.0±13.9 | 33.7±7.33 | 9.32±4.52 |

### Example 3: A single subcutaneous injection study of anti-TSLP antibody in healthy subjects and patients with mild asthma to evaluate safety, tolerability, pharmacokinetics and pharmacodynamics - a randomised, double-blind, dose-escalation, placebo-controlled Phase I clinical trial

1. Investigational medicinal products
   1) Anti-TSLP antibody, prepared according to the method described in WO2022116858, designated as hu179-33 antibody. The heavy and light chain sequences of the anti-TSLP antibody are as disclosed in SEQ ID NO: 9 and 10, formulated as an injection with a concentration of 1 mL: 0.1 g.
   2) Placebo, formulated as an injection with a concentration of: 1 mL.
2. Study methods
   Eligible healthy adults (18-55 years) were randomised in a ratio of 8:2 to receive a single subcutaneous injection of anti-TSLP antibody (five cohorts: 50 mg, 100 mg, 200 mg, 400 mg and 600 mg) or placebo, with a follow-up period ranging from 113 to 253 days for each cohort.
3. Study results
   50 healthy subjects participated and completed the treatment. All treatment-related adverse events (TRAEs) were of mild to moderate severity. The incidence of TRAEs was 45.0% (18/40) in the anti-TSLP antibody group and 20.0% (2/10) in the placebo group. The most common TRAEs (≥5%) in the anti-TSLP antibody group were headache (10.0%), injection site bruising (5.0%) and elevated troponin I (5.0%). Anti-TSLP antibody was absorbed slowly, reaching peak serum concentration (Cmax) at 6.995-17.591 days. The area under the concentration-time curve increased with dose. Within the 50-600 mg dose range, the mean half-life was 69.7-84.9 days. The overall incidence of ADA positivity was similar between the anti-TSLP antibody and placebo groups, and no significant impact on pharmacokinetic characteristics was observed in ADA-positive subjects.

Conclusion: A single subcutaneous injection of anti-TSLP antibody at doses of 50 mg to 600 mg exhibited slightly more than dose-proportional pharmacokinetics in healthy adult subjects, with acceptable safety and tolerability. The overall safety and tolerability of anti-TSLP antibody were good within the 50-600 mg dose range.

### Example 4: Efficacy and safety of anti-TSLP antibody injection in patients with severe uncontrolled asthma - a multicentre, randomised, double-blind, placebo-parallel controlled Phase II clinical study

### 1. Medicinal product information

Anti-TSLP antibody injection: Its antibody sequence corresponds to SEQ ID NO: 9 for the heavy chain, and SEQ ID NO: 10 for the light chain, specification 1 mL: 0.1 g, provided by Jiangsu Hengrui Pharmaceuticals Co., Ltd.

Placebo injection: Specification 1 mL, provided by
Jiangsu Hengrui Pharmaceuticals Co., Ltd.

### 2. Study design:

This is a multicentre, randomised, double-blind, placebo-controlled, parallel-design Phase II clinical trial in patients with severe uncontrolled asthma, requiring medium/high-dose ICS treatment and ≥2 acute asthma attacks in the 12 months prior to randomisation (requiring oral/intravenous corticosteroids). This study plans to enrol 248 subjects (with 50% receiving medium/high-dose ICS before randomisation; and 50% with baseline EOS <300/µL and ≥300/µL respectively), randomised 1:1:1:1 to three treatment regimen groups or placebo group, 62 subjects per group. Subjects in the treatment arms will receive subcutaneous injections of anti-TSLP antibody: Group A 200 mg Q24W, Group B 400 mg Q24W, Group C 400 mg Q12W. The placebo group will receive placebo subcutaneously every 12 weeks.

### Study procedure:

The study consists of five periods: Screening period, run-in period, baseline period, treatment period, and follow-up period.

**Screening period (W-4~W-3):** A total of 248 subjects with severe uncontrolled asthma will be screened, regardless of sex, with 40% ≤FEV₁% predicted <80% and ≥2 acute exacerbations within 12 months prior to randomisation (requiring oral/intravenous corticosteroids). Eligible subjects will be trained on use of home peak flow meter, equipped with electronic patient-reported outcome (ePRO) system and instructed accordingly, including inhaled medication usage.

**Run-in period (W-2~W-1):** Subjects must record daily asthma diary in the ePRO system, fill in the patient log, measure PEF twice daily (morning and evening) using the home peak flow meter, and record in the ePRO system. Run-in visit will assess subject's technical ability and compliance with home peak flow meter and ePRO system. From W-1, subject's compliance with peak flow meter and ePRO system is calculated, requiring ≥70% compliance within 7 days prior to randomisation.

**Baseline period:** Subjects who pass screening will undergo further assessments and compliance checks during baseline. Those meeting all inclusion criteria and none of the exclusion criteria will receive randomisation and medication number during baseline, and will be randomised 1:1:1:1 into treatment or placebo groups.

### Treatment period (W1~W48):

Treatment regimens for each group

| Grouping | Dosing regimen |
|---|---|
| Group A | W1D1 subcutaneous injection: 2× Anti-TSLP antibody injection 100 mg (1 mL) + 2× Placebo 1 mL |
| | W13D1 subcutaneous injection: 4× Placebo 1 mL |
| | W25D1 subcutaneous injection: 2× Anti-TSLP antibody injection 100 mg (1 mL) + 2× Placebo 1 mL |
| | W37D1 subcutaneous injection: 4× Placebo 1 mL |
| Group B | W1D1 subcutaneous injection: 4× Anti-TSLP antibody injection 100 mg (1 mL) |
| | W13D1 subcutaneous injection: 4× Placebo 1 mL |
| | W25D1 subcutaneous injection: 4× Anti-TSLP antibody injection 100 mg (1 mL) |
| | W37D1 subcutaneous injection: 4× Placebo 1 mL |
| Group C | W1D1, W13D1, W25D1, W37D1 subcutaneous injection: 4× Anti-TSLP antibody injection 100 mg (1 mL) |
| Placebo group | W1D1, W13D1, W25D1, W37D1 subcutaneous injection: 4× Placebo 1 mL |

**Follow-up period (W49~W72):** Subjects will enter the follow-up period after the end of the treatment period. During the follow-up, subjects will be contacted proactively. Information collection must be conducted at least once every 4 weeks to capture safety events in the clinical trial.

### Primary endpoint and key estimand:

The primary endpoint is the annualised asthma exacerbation rate (AAER) at Week 48. Annualised asthma exacerbation rate (AAER) = Number of acute asthma attacks * 365.25 / duration of follow-up (days)

The five attributes of the key estimand are defined as follows:
1. Treatment: Experimental groups include anti-TSLP antibody (Group A 200 mg Q24W, Group B 400 mg Q24W, Group C 400 mg Q12W) + maintenance therapy; control group includes placebo + maintenance therapy;
Maintenance therapy includes medium/high-dose inhaled corticosteroids (ICS) + at least one other asthma control medication, as defined in inclusion criteria 4 and 5.
2. Population: Patients with severe uncontrolled asthma;
3. Variable (endpoint): Annualised asthma exacerbation rate (AAER) at Week 48;
4. Intercurrent events and strategies:

| Intercurrent event | Strategy |
|---|---|
| 1. Treatment discontinuation | Post-discontinuation data will be used for AAER analysis (treatment policy strategy) |

5. Population-level summary: Ratio of AAER between the treatment and control groups (to be estimated using a negative binomial regression model).

### Secondary endpoints:

Change from baseline in pre-/post-bronchodilator (Pre-/Post-BD) forced expiratory volume in 1 second (FEV1), forced vital capacity (FVC), and peak expiratory flow (PEF) at each assessment timepoint.

Change from baseline in fractional exhaled nitric oxide (FeNO), blood eosinophil count (EOS) and serum immunoglobulin E (IgE) at each assessment timepoint.

Change from baseline in Asthma Control Questionnaire (ACQ-6) score at each assessment timepoint.

Change from baseline in Asthma Quality of Life Questionnaire (AQLQ) score at each assessment timepoint.

Annualised rate of acute asthma exacerbations leading to hospitalisation at Week 48.

Change from baseline in weekly mean asthma symptom diary score.

Proportion of subjects experiencing acute asthma attacks.

Change from baseline in weekly rescue medication use.

Change from baseline in weekly mean morning and evening PEF.

Change from baseline in weekly mean number of nocturnal awakenings.

Time to first acute asthma attack.

Change in 22-item Sino-Nasal Outcome Test (SNOT-22) score at each assessment timepoint in pataients with nasal polyps.

Incidence of composite exacerbation endpoint (CompEx).

### Safety endpoints:

Physical examination, vital signs, ECG, laboratory tests and adverse events/serious adverse events.

### Pharmacokinetic (PK) and immunogenicity endpoints:

PK: Serum drug concentration of anti-TSLP antibody.

Immunogenicity: Incidence, time of onset, duration and titre of anti-drug antibodies (ADA).

### 3. Inclusion criteria

Subjects must meet all of the following criteria to be eligible for this study:
(1) Age ≥18 years and ≤75 years, male or female;
(2) Body weight ≥40 kg;
(3) Diagnosed with asthma as per the 2020 version of the Guidelines for the Prevention and Treatment of Bronchial Asthma, with a history of at least 1 year;
(4) Received medium/high-dose inhaled corticosteroid (ICS) therapy for at least 6 months prior to screening and remained stable for 3 months before randomisation;
   a. High-dose ICS is defined as a total daily dose >500 µg of fluticasone propionate via dry powder inhaler (DPI) or pressurised metered-dose inhaler (pMDI), or equivalent;
   b. Medium-dose ICS is defined as a total daily dose >250 µg and ≤500 µg of fluticasone propionate via DPI or pMDI, or equivalent;
   c. Equivalent ICS doses will be determined according to the 2020 version of the asthma treatment guidelines;
(5) Concomitant use of at least one other asthma control medication [including long-acting β2 agonist (LABA), long-acting muscarinic antagonist (LAMA), leukotriene receptor antagonist (LTRA), maintenance oral corticosteroids (OCS; maximum of 10 mg daily or 20 mg every other day of prednisone for maintenance), or equivalent, excluding theophyllines], with stable use 3 months prior to randomisation;
(6) During the screening and baseline periods, the pre-bronchodilator FEV₁ is ≥40% and <80% of the predicted value;
(7) ACQ-6 score ≥1.5 during screening;
(8) At least 2 acute asthma exacerbation events within 12 months prior to randomisation, with at least 1 occurring during medium/high-dose ICS treatment. Acute asthma attack event is defined as systemic corticosteroid use or increased dose of maintenance systemic corticosteroids for ≥3 days;
(9) Female subjects of childbearing potential and male subjects with partners of childbearing potential must agree to have no plans for conception and use appropriate contraception from signing the informed consent form until 19 months after the last dose;
(10) Voluntarily sign the informed consent form to participate in this study.

### 4. Exclusion criteria:

Subjects will be excluded from the study if any of the following criteria are met:
1. Clinically significant major pulmonary diseases, including but not limited to tuberculosis (including old/latent TB), bronchiectasis, atelectasis, interstitial lung disease, allergic bronchopulmonary aspergillosis, chronic obstructive pulmonary disease (COPD), pulmonary vascular disease, tumours, etc.;
2. Concomitant diseases other than asthma that may affect lung function, including but not limited to clinically significant pleural diseases, mediastinal diseases, diaphragmatic disorders, myasthenia and thoracic deformities;
3. Other eosinophil-related disorders, including but not limited to hypereosinophilic syndrome (HES), eosinophilic granulomatosis with polyangiitis (EGPA), eosinophilic oesophagitis, etc.;
4. Immunodeficiency disorders;
5. Clinically significant, unstable or uncontrolled serious cardiovascular or cerebrovascular diseases, including but not limited to myocardial infarction, unstable angina, heart failure, stroke and subarachnoid haemorrhage, etc.;
6. Uncontrolled hypertension (systolic BP ≥180 mmHg and/or diastolic BP ≥110 mmHg during screening);
7. Uncontrolled diabetes (fasting plasma glucose >11.1 mmol/L during screening);
8. Clinically significant infections within 4 weeks prior to randomisation, including but not limited to upper or lower respiratory tract infections;
9. Major surgery within 3 months prior to randomisation or planned surgery during the study period, or any treatment deemed by the investigator to potentially affect subject evaluation;
10. Known parasitic infection within 6 months prior to randomisation;
11. Diagnosis of malignancy within 5 years prior to randomisation (except malignancies with low risk of metastasis or mortality, e.g.,adequately treated basal cell carcinoma of the skin or cervical carcinoma in situ);
12. Abnormal laboratory results during screening and baseline:
   White blood cell count (WBC) <3.0×10⁹/L;
   Haemoglobin (Hb) ≤90 g/L;
   Platelet count (PLT) <100×10⁹/L;
   Alanine aminotransferase (ALT) >3×ULN (upper limit of normal);
   Aspartate aminotransferase (AST) >3×ULN;
   Total bilirubin (TBIL) >ULN;
   Prothrombin time (PT) >ULN + 3s;
   Creatinine (Cr) >1.5×ULN; or glomerular filtration rate <50 mL/min (calculated using modified MDRD formula for Chinese population: eGFR [mL/min/1.73m²] = 175 × Scr^{-1.234} × [age]^{-0.119} × [female × 0.79], serum creatinine (Scr): (mg/dL), age: years, Scr 1 mg/dL = 88.4 µmol/L);
13. Active hepatitis B infection (HBsAg positive with peripheral HBV DNA ≥1×10³ IU [or copies]/mL), or positive for hepatitis C antibody, HIV antibody, or syphilis antibody;
14. QTc prolongation on ECG at screening or baseline (male >450 ms, female >470 ms) or any other clinically significant abnormal findings deemed to pose significant safety risk to the subject;
15. Use of non-selective beta-blockers (e.g., propranolol, carvedilol) within 1 week prior to randomisation;
16. Use of 5-lipoxygenase inhibitors (e.g. zileuton) or phosphodiesterase-4 inhibitors (e.g. roflumilast) within 1 week prior to randomisation;
17. Live vaccines, attenuated live vaccines, or viral vector vaccines received within 4 weeks prior to randomisation;
18. Infusion of blood products or immunoglobulin within 4 weeks prior to randomisation;
19. Allergen immunotherapy within 8 weeks prior to randomisation;
20. Use of systemic immunosuppressants (excluding systemic corticosteroids used for asthma treatment), immunomodulators, biologics, or Th₂ cytokine inhibitors within 12 weeks prior to randomisation or within 5 drug half-lives (as specified in the package insert); if the half-life is unknown, 12 weeks prior to randomisation shall apply), including but not limited to methotrexate, cyclosporine, interferon-α, anti-interleukin-5 (IL-5) monoclonal antibody, anti-interleukin-4 receptor (IL-4R) monoclonal antibody, anti-TSLP monoclonal antibody, anti-IgE monoclonal antibody and pranlukast;
21. Bronchial thermoplasty within 1 year prior to randomisation;
22. Current smoker or quit smoking less than 6 months before screening and/or positive urine cotinine test at screening, or smoking history ≥10 pack-years (pack-years = number of years smoked × number of packs per day);
23. History of drug abuse, substance abuse and/or excessive alcohol use within 1 year prior to screening. Excessive alcohol use is defined as daily alcohol consumption ≥15 g for adults, equivalent to 450 mL of beer, 150 mL of wine, 50 mL of 38% spirits, or 30 mL of high-proof spirits;
24. Known allergy to any biologics or any history of hypersensitivity that the investigator considers unsuitable for study participation;
25. Pregnancy (including positive pregnancy test at screening or baseline), breastfeeding, or planned pregnancy during the study period;
26. Participation in another clinical trial involving investigational medicinal product with active ingredients within 4 weeks prior to screening or still within 5 half-lives of previous investigational product at screening (whichever is longer);
27. Other medical or social reasons deemed unsuitable for study participation by the investigator.

## Claims

1. A method for treating a subject suffering from asthma, and/or reducing the frequency and/or severity of asthma exacerbations in the subject, and/or reducing the annualised asthma exacerbation rate in the subject, and/or reducing the subject's ACQ-6 score, comprising: 1) administering to the patient at least one anti-TSLP antibody or antigen-binding fragment thereof at dosing intervals not shorter than 10 weeks;
Preferably, the dosing interval is selected from 10, 12, 14, 16, 18, 20, 22, 24, 26, 28 weeks, or longer;
More preferably, the dosing interval is selected from 12, 16, 20, 24 weeks or longer;
Most preferably, the dosing interval is 12 weeks or 24 weeks;
or 2) administering once every 10, 12, 14, 16, 18, 20, 22, 24, 26, 28 weeks;
More preferably, administering once every 12, 16, 20, 24 weeks;
Most preferably, administering once every 12 weeks or 24 weeks.

2. The method according to claim 1, wherein the dosage of the anti-TSLP antibody or antigen-binding fragment thereof is up to about 600 mg;
Preferably 50-600 mg, 100-500 mg or 150-450 mg;
More preferably selected from about 100 mg, about 150 mg, about 200 mg, about 250 mg, about 300 mg, about 350 mg, about 400 mg, about 450 mg or about 600 mg;
Most preferably about 200 mg or about 400 mg.

3. The method according to claim 1 or 2, wherein the dosing regimen of the anti-TSLP antibody or antigen-binding fragment thereof is selected from 200 mg Q24W, 400 mg Q24W, or 400 mg Q12W.

4. The method according to any one of claims 1-3, wherein the anti-TSLP antibody or antigen-binding fragment thereof is administered for a duration of at least 2 months, 3 months, 4 months, 5 months, 6 months, 8 months, 10 months, 12 months or longer.

5. The method according to any one of claims 1-4, wherein the anti-TSLP antibody or antigen-binding fragment thereof is administered subcutaneously or intravenously, preferably by subcutaneous injection.

6. The method according to any one of claims 1-5, wherein the subject has allergic asthma or non-allergic asthma.

7. The method according to any one of claims 1-5, wherein the subject has mild asthma.

8. The method according to any one of claims 1-5, wherein the subject has moderate asthma.

9. The method according to any one of claims 1-5, wherein the subject has moderate to severe asthma or severe asthma.

10. The method according to claim 8 or 9, wherein the subject has uncontrolled asthma, preferably severe uncontrolled asthma.

11. The method according to any one of claims 1-10, wherein the subject has eosinophilic asthma or non-eosinophilic asthma.

12. The method according to claim 11, wherein the subject has a high eosinophil count at the start of treatment or at diagnosis, preferably ≥250 cells/µL, more preferably ≥300 cells/µL.

13. The method according to claim 11, wherein the subject has a low eosinophil count at the start of treatment or at diagnosis, preferably <300 cells/µL, more preferably <250 cells/µL.

14. The method according to any one of claims 1-13, wherein the anti-TSLP antibody or antigen-binding fragment thereof comprises a heavy chain variable region and a light chain variable region, wherein:
the heavy chain variable region comprises HCDR1, HCDR2 and HCDR3 as shown in SEQ ID NO: 1, SEQ ID NO: 2 and SEQ ID NO: 3, respectively; and
the light chain variable region comprises LCDR1, LCDR2 and LCDR3 as shown in SEQ ID NO: 4, SEQ ID NO: 5 and SEQ ID NO: 6, respectively.

15. The method according to any one of claims 1-14, wherein the anti-TSLP antibody comprises a heavy chain variable region and a light chain variable region, the heavy chain variable region comprises an amino acid sequence as shown in SEQ ID NO: 7 or having 90% identity thereto, and the light chain variable region comprises an amino acid sequence as shown in SEQ ID NO: 8 or having 90% identity thereto.

16. The method according to any one of claims 1-15, wherein the anti-TSLP antibody comprises an immunoglobulin constant region;
Preferably, comprises an Fc region derived from human IgG;
Preferably, comprises an Fc region derived from human IgG1, IgG2, IgG3 or IgG4.

17. The method according to any one of claims 1-16, wherein the anti-TSLP antibody comprises a heavy chain and a light chain, the heavy chain comprises an amino acid sequence as shown in SEQ ID NO: 9 or having 90% identity thereto, and the light chain comprises an amino acid sequence as shown in SEQ ID NO: 10 or having 90% identity thereto.

18. The method according to any one of claims 1-17, wherein the subject also maintains background therapy as maintenance treatment.

19. The method according to claim 18, wherein the background therapy includes at least one of the following:
a) glucocorticoids, preferably inhaled corticosteroids (ICS) or oral glucocorticoids;
b) bronchodilators, preferably long-acting β2 agonists (LABA), short-acting β2 agonists (SABA), anticholinergic drugs;
c) long-acting muscarinic receptor antagonists (LAMA);
d) leukotriene receptor antagonists (LTRA);
e) cromolyn;
f) antihistamines;
g) anti-leukotriene agents;
h) PDE-4 inhibitors.

20. Use of an anti-TSLP antibody or antigen-binding fragment in combination with other asthma treatment drugs in the preparation of a medicament for treating a subject suffering from asthma, or reducing the frequency and/or severity of asthma exacerbations in the subject, or reducing the annualised asthma exacerbation rate in the subject, or reducing the subject's ACQ-6 score, wherein the anti-TSLP antibody or antigen-binding fragment thereof is as defined in any one of claims 1-17, and the other asthma treatment drugs are selected from the background therapy drugs as defined in claim 18 or 19.
